# EUROPEAN PATENT APPLICATION

(11) **EP 2 404 993 A1**
(43) Date of publication of application: **11.01.2012**
(21) Application number: 10169151.7
(22) Date of filing: 09.07.2010
(51) Int. Cl.: C12N 5/00, A61Q 19/08, C12N 5/071, A61L 27/38

(54) **Alginate filler including eukaryotic cells**

(71) Applicant: Merz Pharma GmbH & Co. KGaA, 60318 Frankfurt Main (DE)
(72) Inventor: Liebetrau, Wolfgang, Dr., 61169 Friedberg (DE)
(74) Representative: Hüttermann, Aloys

(57) **Abstract**

A composition for use as a dermal filler, and a method for improving skin imperfections are disclosed, the composition comprising an alloplastic injectable suspension comprising beads based on a biocompatible material and a physiologically acceptable suspending agent, wherein the beads include eukaryotic cells synthesizing and/or secreting one or more constituents of the dermal extracellular matrix, said eukaryotic cells being encapsulated by a sheath of said biocompatible material, and the method comprises intradermal and/or subdermal under-injection of said composition.

## Description

### FIELD OF INVENTION

The present invention relates a composition for use as a dermal filler, the composition comprising an alloplastic injectable suspension comprising beads based on a biocompatible material and a physiologically acceptable suspending agent, wherein the beads include eukaryotic cells synthesizing and/or secreting one or more constituents of the dermal extracellular matrix, said eukaryotic cells being encapsulated by a sheath of said biocompatible material. The present invention also relates to a method for improving imperfections of the skin, the method comprising intradermal and/or subdermal under-injection of an alloplastic injectable suspension, the suspension comprising beads based on a biocompatible material and a physiologically acceptable suspending agent, said beads include eukaryotic cells synthesizing and/or secreting one or more constituents of the dermal extracellular matrix, said eukaryotic cells being encapsulated by a sheath of said biocompatible material.

### BACKGROUND OF THE INVENTION

The skin is a soft outer covering of an animal, in particular a vertebrate. In mammals, the skin is the largest organ of the integumentary system made up of multiple layers of ectodermal tissue. The skin is an anatomical barrier which protects one from the environment. The skin assists one in controlling the body temperature, the electrolyte balance and the evaporation of moisture. Moreover, the skin contains a large number of nerve receptors enabling one to sensate heat and cold, touch, pressure, vibration and injuries.

The skin changes as one gets older. Most notable changes of the skin are the appearance of wrinkles, age spots and dryness. The skin also becomes thinner and loses fat, making it less plump and smooth. It might take longer for injuries to heal, too.

The skin and its appearance change due to various factors, namely intrinsic factors and external factors. Intrinsic factors are genetic make-up, biosynthesis of collagen and elastin being slowed down. Dead skin cells are not shed as quickly and turnover of new skin cells is slightly decreased. These processes are known as "intrinsic aging" or the natural aging process. In addition, a number of external factors act together with the normal aging process of the skin leading to premature aging of skin. Most prominent external factors are exposure to sunlight. Other external factors are for example repetitive facial expressions, gravity, sleeping positions and smoking, climate, exposures to industrial and household chemicals, indoor heating, clothing, allergies to plants, other allergies. Many other common exposures can also cause skin changes.

The changes of skin are among the most visible signs of aging. Evidence of increasing age includes wrinkles and sagging skin. Dry skin that may itch and an inability to sweat sufficiently to cool the skin are further changes of the skin upon aging.

The skin is generally divided into three main layers. The outer layer (epidermis) contains skin cells, pigment, and proteins. The middle layer (dermis) contains blood vessels, nerves, hair follicles, and oil glands. The dermis provides nutrients to the epidermis. The inner layer underneath the dermis (the subcutaneous layer) contains sweat glands, some hair follicles, blood vessels, and fat. Each layer also contains connective tissue with collagen fibers to give support and elastin fibers to provide flexibility and strength.

With aging, the outer skin layer (epidermis) thins even though the number of cell layers remains unchanged. The number of pigment-containing cells (melanocytes) decreases, but the remaining melanocytes increase in size. Aging skin thus appears thinner, more pale, and translucent. Large pigmented spots (called age spots, liver spots, or lentigos) may appear in sun-exposed areas.

Changes in the connective tissue reduce the skin's strength and elasticity. This is known as elastosis and is especially pronounced in sun-exposed areas (solar elastosis). Elastosis produces the leathery, weather-beaten appearance common to farmers, sailors, and others who spend a large amount of time outdoors.

Sebaceous glands produce less oil as one ages. Men experience a minimal decrease, usually after the age of 80. Women gradually produce less oil beginning after menopause. This can make it harder to keep the skin moist, resulting in dryness and itchiness.

The subcutaneous fat layer, which provides insulation and padding, thins. This increases the risk of skin injury and reduces the ability to maintain body temperature.

The sweat glands produce less sweat. This makes it harder to keep cool, and the risk for becoming overheated or developing heat stroke increases.

Aging skin repairs itself more slowly than younger skin. Wound healing may be up to 4 times slower. This contributes to pressure ulcers and infections. Diabetes, blood vessel changes, lowered immunity, and similar factors also affect healing.

Many products claim to revitalize aging skin or reduce wrinkles, but the Food and Drug Administration has approved only a few for sun-damaged or aging skin. Various treatments soothe dry skin and reduce the appearance of age spots.

Recently, a suspension of human dermal fibroblasts (HDFs) in a cell storage medium is commercialized in United Kingdom under the trade name VAVELTA® for treating skin imperfections. The suspension of HDFs is directly injected into the target area using a fine gauge needle, and the collagen secreted by the HDFs within the dermis is considered to restructure and repair the extracellular matrix and thereby improves the structure, function and appearance of skin damaged by scarring or the aging process. However, repeated injections may have to be given as HDFs injected into the dermis appear to be active for a few weeks only.

Thus, there is an ongoing demand for improvements in improving imperfections of the skin, i.e. an improvement in the appearance, structure and/or function of skin.

WO 00/47237 A1 discloses a drug delivery device comprising encapsulated cells which contain an expression cassette. The cells within this drug delivery device contain a heterologous polynucleotide for expressing a protein or polypeptide having metabolic activity such that administration of said cells might control, prevent or treat a metabolic disorder. In an approach to prevent rejection of the foreign cells and to protect these cells from the immune system, it is proposed to encapsulate those cells, preferably in a capsule that is double layered. However, neither a specific method of encapsulating cells nor any materials said capsules may comprise or consist of are explicitly disclosed.

### OBJECTS OF THE INVENTION

It has surprisingly been found that life and activity of HDFs can be significantly extended when these cells were encapsulated into an alginate sheath before being administered intradermally.

Based on these results, it is a first aspect of the present invention to provide compositions for improving the appearance, structure and/or function of skin, e. g. for use as dermal filler, wherein said compositions comprise an alloplastic injectable suspension comprising beads based on a biocompatible material and a physiologically acceptable suspending agent, wherein the beads include eukaryotic cells synthesizing and/or secreting one or more constituents of the dermal extracellular matrix, said eukaryotic cells being encapsulated by a sheath of said biocompatible material.

In a second aspect, the present invention provides a method for improving the appearance, structure and/or function of skin, the method comprising intradermal and/or subdermal under-injection of an alloplastic injectable suspension as dermal filler, the suspension comprising beads based on a biocompatible material and a physiologically acceptable suspending agent, said beads include eukaryotic cells synthesizing and/or secreting one or more constituents of the dermal extracellular matrix, said eukaryotic cells being encapsulated by a sheath of said biocompatible material.

In yet another aspect, the present invention pertains to the use of compositions comprising eukaryotic cells synthesizing and/or secreting constituents of the extracellular dermal matrix wherein said cells are encapsulated in a biocompatible and/or biodegradable polymer sheath for improving the appearance, structure and/or function of skin.

The use according to the invention is suitable in particular for treatment of wrinkles e.g. of forehead wrinkles, anger wrinkles, worry wrinkles, slack eyelids, crows' feet, nasolabial wrinkles and for under-injection of lips and for treatment of wrinkles in the hand region, Cellulite and decoltee.

It is an further object of the present invention to provide a novel filler exhibiting a long-lasting effect and much less side effects.

### SUMMARY OF THE INVENTION

According to the first aspect of the invention, a composition is provided wherein eukaryotic cells are incorporated or encapsulated into a sheath. Said eukaryotic cells are cells which synthesize and/or secrete a constituent of the dermal extracellular matrix.

The extracellular matrix is composed of a variety of proteins and polysaccharides that are secreted locally and assembled into an organized meshwork in close association with the surface of the cells that produce those proteins and/or polysaccharides. Usually the macromolecules of the extracellular matrix are secreted by fibroblasts.

The extracellular matrix is an intricate network of macromolecules which is present in the extracellular space of tissues. The macromolecules constituting the extracellular matrix are mainly synthesized locally by the cells within the matrix. The main classes of extracellular macromolecules that make up the matrix are fibrous proteins and polysaccharide chains of the class of glycosaminoglycans, which are usually covalently linked to proteins. Glycosaminoglycans include hyaluronan, chondroitin sulfate, dermatan sulfate, heparin sulfate, heparin and keratin sulfate. Proteoglycans comprise a core protein and a polysaccharide chain. Examples of proteoglycans of the extracellular matrix are aggrecan, betaglycan decorin, perlecan, serglycin and syndecan-l. Fibrous proteins of the extracellular matrix include, for example, collagen and elastin which have mainly structural functions, and fibronectin and laminin which have mainly adhesive functions.

According to another embodiment of the first aspect of the present invention, the eukaryotic cells which are encapsulated into a sheath are fibroblasts. According to another embodiment, the eukaryotic cells are human dermal fibroblasts.

According to an embodiment of the present invention, the eukaryotic cells synthesize and/or secrete at least one macromolecule of the extracellular matrix, wherein said macromolecules are selected from the group consisting of polysaccharides and proteins.

The polysaccharides are glycosaminoglycans. Glycosaminoglycans are usually covalently linked to proteins in form of proteoglycans. Glycosaminoglycans are composed of repeating disaccharide units, one of the two saccharides being an amino sugar, i.e. N-acetylglucosamine or N-acetylgalactosamine, which is usually sulfated. According to an embodiment of the present invention, the glycosaminoglycans are selected from the group consisting of hyaluronan, chondroitinsulfate, dermatan sulfate, heparin sulfate, heparin and keratin sulfate.

Except for hyaluronan, all glycosaminoglycans are covalently attached to protein, a so -called core protein, in form of proteoglycans. According to another embodiment of the first aspect of the present invention, the eukaryotic cells synthesize at least one proteoglycan which is selected from the group consisting of aggrecan, betaglycan, decorin, perlecan, serglycin and syndecan-l.

Pursuant to another and/or additional embodiment of the first aspect of the present invention, the eukaryotic cells being encapsulated, synthesize and/or secrete a protein of the dermal extracellular matrix. Said protein of the extracellular matrix may be a fibrous protein having mainly structural function and, for example, be selected from the group consisting of such as collagen and elastin. According to an embodiment of the present invention, the collagen is selected from the group consisting of collagen type I, collagen type III, collagen type IV, collagen type V, and collagen precursors such as pro-alpha-chains of collagen, procollagen triple helices, procollagen molecules and collagen fibrils having a diameter of about 10 nm to 300 nm..

The protein of the dermal extracellular matrix that may be synthesized and/or secreted by the encapsulated eukaryotic cells may have mainly adhesive function and for example be selected from the group consisting of fibronectin and laminin.

The eukaryotic cells may either be naturally occurring cells, i. e. cells obtained from natural sources and not genetically modified. However, it is also possible to utilize genetically modified eukaryotic cells.

According to the first aspect of the present invention, the eukaryotic cells are encapsulated in a polymeric sheath. Said polymeric sheath is based on a biocompatible and/or biodegradable polymer. Suitable polymers for the sheath are for example, polylactide, chondroitin sulfate, polyesters, polyethylene glycols, polycarbonates, polyvinyl alcohols, polyacrylamides, polyamides, polyacrylates, polyetheresters, polymethacrylates, polyurethanes, polycaprolactone, polyphophazenes, polyorthoesters, polyglycolides, copolymers of lysine and lactic acid, copolymers of lysine-RGD and lactic acid. According to other embodiments, the biocompatible and/or biodegradable polymer is selected from the group of naturally derived materials such as chitosan, alginates, collagens, hyaluronic acid, pectin, gellan and carrageenan.

In another embodiment of the first aspect of the present invention, the polymeric sheath wherein the eukaryotic cells are incorporated is an alginate. In an embodiment, the eukaryotic cells are incorporated into a cross-linked alginate. Said cross-linked alginate may be selected from the group consisting of Ca²⁺-alginate, Ba²⁺-alginate, and mixtures thereof, i. e. alginates, wherein said alginate is cross-linked by calcium ions, barium ions or a mixture of calcium ions and barium ions.

In a further embodiment of the invention the polymeric sheath is a mixture of different polymers.

Wrinkles that may be treated by employing the filler according to the instant invention include, but are not limited to, frown lines, medium depth wrinkles, such as nasolabial folds, lip augmentation, forehead wrinkles, glabellar lines, obvious mild to moderate nasal furrows and cheek wrinkles, crow's feet, perioral wrinkles, and acne scars.

The present invention further pertains to a method of treating a medical condition, including lipoatrophy, gastroesophageal reflux disease (GERD), urine incontinence, vesico ureteral reflux (VUR), or a psychological condition caused by the appearance of an aesthetic deficiency, including, but not limited to, frown lines, medium depth wrinkles, such as nasolabial folds, lip augmentation, forehead wrinkles, glabellar lines, obvious mild to moderate nasal furrows and cheek wrinkles, crow's feet, perioral wrinkles and acne scars, wherein said method comprises a step of administering a filler as claimed in the present invention to a patient in need thereof.

The present invention further pertains to a method of using a filler according to the present invention in plastic, cosmetic, dental or general surgery, in ophthalmology, in orthopedics, as products for preventing tissue adhesions, or in urology, wherein said method comprises a step of administering a filler as claimed in the present invention to a patient in need thereof.

In one embodiment, the aqueous solution containing divalent cations further comprises one or more active pharmaceutical ingredient selected from the group of anesthetics, analgesics, anti-microbials, anti-inflammatory drugs, growth factors, hormones, cosmeceuticals, vitamins, nutrients, stimulants, steroids, vasoconstrictors, anti-thrombotic agents, anti-coagulation agents, tranquilizers, muscle relaxants, antifungals, lipolytic agents and biorejunevation agents.

Further, the present invention pertains to a kit comprising a filler as provided herein and an injection device. The injection device could be a prefilled syringe or an electronic injection device.

Further, the present invention pertains to an injection device comprising the filler provided herein. The injection device could be a prefilled syringe or an electronic injection device.

### DETAILED DESCRIPTION OF THE INVENTION

The term "alloplastic" refers to non-endogenous material or compositions, i.e. to composition comprising non-endogeneous materials or components. An alloplastic composition may comprise beads that are based on a biocompatibel and/or biodegradable polymer. Even if the beads encapsulate endogeneous cells of a patient, hence cells that were obtained form a person and processed to become encapsulated in beads, a suspension comprising said beads is an alloplastic suspension for the present disclosure, because the polymer is a non-endogenous naturally or synthetic material.

The term "bead" refers to a substantially spherical or ellipsoid particle made of a a porous biocompatible and/or biodegradable polymeric material, wherein the size of the particle is between 10 µm and 2,000 µm.

The term "alginate" refers to a naturally occurring anionic unbranched polysaccharide which is isolated from marine brown algae. It is built up from homopolymeric groups of -D-mannuronic acid and -L-guluronic acid, separated by heteropolymeric regions of the two acids. The technical-grade alginates nowadays already obtained in large amounts are employed in industry (e.g. papermaking) and as foodstuffs additive. However, they are also being employed to an increasing extent in pharmacy, medicine and biotechnology. They are routinely used as a constituent of wound dressings. Alginates also have been and are being employed in a whole series of "tissue engineering" and "drug delivery" projects. The decisive property of alginates for use in biotechnology and in medicine is their capacity for ionotropic gel formation. The alkali metal salts of alginates are water-soluble, while the salts of alginates with most di- or polyvalent cations form insoluble gels in aqueous solution (so-called hydrogels). The wide range of physical variation of the alginates is determined by a number of factors: viscosity (or molar mass distribution, respectively), concentration, ratio of monomers and affinity of the cation employed for the crosslinking. It is thus known e.g. that alginates crosslinked with calcium form less stable hydrogels than alginates which are crosslinked with barium. Not all alginates are suitable for the use described here, since they can contain impurities which after implantation into humans can cause immune defence reaction, such as, for example, fibrosis or inflammatory reactions.

A highly pure alginate which is to be used according to an embodiment of the present invention and which can be isolated by using homogeneous algal raw material and standardized processes. The biocompatibility requirements are thereby met.

Purified alginates having an average molar mass of from 20 kDa to 10,000 kDa can be used, and according to an embodiment of the present invention, the molar mass is between 100 kDa and 1,200 kDa. The viscosity of a 0.1 % strength (w/v) prepared aqueous alginate solution of the alginate to be used can be between 3 and 100 mPa s, and is between 10 and 40 mPa s in another embodiment of the present invention. The concentration of the alginate for the preparation of the alginate solution to be used is between 0.1 and 4% (w/v), and is between 0.4 and 1 % (w/v) in another embodiment of the present invention. In one embodiment, the viscosity of a 0.6% strength (w/v) prepared aqueous alginate solution in 0.9% NaCl of the alginate to be used is between 600 and 700 mPa·s.

For preparation of the beads, the geometric form thereof is first shaped from the alginate. In order to produce spherical beads, an alginate solution (e.g. potassium alginate or sodium alginate in physiological saline solution) is expediently formed into drops in a precipitating bath with dissolved crosslinking agent. According to an embodiment of the invention, the crosslinking agent consists of divalent cations, e.g. dissolved calcium or barium (5-100 mM). In yet another embodiment of the invention, the precipitating bath additionally also contains a buffer substance (e.g. histidine) and sodium chloride (e.g. 290 mOsmol). Air-charged spray nozzles or other encapsulation and drop formation methods corresponding to the prior art are suitable for the preparation of spherical beads. During the preparation process, additional substances (e.g. vitamins, adhesion proteins, antiinflammatory substances, antibiotics, growth factors, hormones, nutrients, marker substances, vital cells) can also be introduced into the alginate beads in the manner known in the prior art. After the preparation, several washing steps with a physiological saline solution or another suitable washing solution follows. The beads are separated from the precipitating and washing baths with a centrifuge or other suitable methods according to an embodiment of the invention. Beads of crosslinked alginate are not rigid or solid structures, but are highly flexible and elastic, but nevertheless dimensionally stable.

As an alternative, a JetCutter device (geniaLab, Braunschweig, Germany) may be employed to produce beads having cells encapsulated therein. In this method, bead generation is achieved by cutting a solid jet of fluid, preferably a viscous fluid, coming out of a nozzle by means of rotating cutting wires into cylindrical segments which then form beads due to surface tension on their way to a hardening device.

The size of the beads depends on the drop formation method and on the viscosity (or on the molar mass distribution of the alginate) and the concentration of the alginate solution. Beads having a diameter of from 10 µm to 2,000 µm can be used, Beads having a diameter of from 100 µm to 400 µm are used in another embodiment of the invention. The preparation and transfer to containers of the alginate solution or of the beads of alginate are carried out under sterile conditions and/or the products are subjected to final sterilization using a suitable method corresponding to the prior art (e.g. gamma sterilization). Both the alginate solution and the beads of crosslinked alginate are stable to storage in the frozen state.

The sheath including the eukaryotic cells comprises pores. The size of the pores within the sheath depends on the molecules to be secreted by the eukaryotic cells encapsulated by said sheath. The size of the pores within the sheath is - in average - at least 10 nm in diameter, at least 20 nm in diameter in another embodiment, at least 25 in diameter in a further embodiment, at least 50 nm in diameter in yet another embodiment and at least 100 nm in diameter in still another embodiment. The size of the pores shall not exceed - in average - 300 nm in diameter, does not exceed 200 nm in diameter in an embodiment of the invention, does not exceed 150 nm in diameter in another embodiment of the invention, and does not exceed 100 nm in diameter in yet another embodiment of the invention.

In an embodiment of the invention, a bead contains from about 40 to about 1,000 eukaryotic cells. In another embodiment or the invention, a bead contains from about 60 to about 500 eukaryotic cells and in yet another embodiment from about 80 to about 120 eukaryotic cells. However, beads designed to contain larger numbers of cells can also be used for the composition of the present invention, for example more than 1,000 cells, or more than 10,000 cells, or more than 50,000 cells, or more than 100,000 cells.

The composition of the first aspect of the present invention may comprise the encapsulated cells in form of a suspension. In an embodiment of the invention, the suspension is injectable. As used herein, "injectable" means that the composition of the present invention can be readily injected into a target area of the body of a living subject such as mammals, using any injection means including, but not limited to, needles, syringes, and the like.

According to the instant invention the filler may comprise a medium in which the beads are suspended. Said medium may be sterile water, phosphate-buffer saline (PBS), ringer solution, isotonic saline solution (0.9%), trometamol, citrate, carbonate, acetate, borate, amino acid, diethylamine, glucono delta lactone, glycine, lactate, maleic, methanesulfonic, monoethanolamine, tartrate buffer of choice or any combination thereof.

According to an embodiment of the invention, the composition according to the first aspect of the invention is used for treating skin wrinkles by intra- or subdermal under-injection of the areas of skin affected. According to an embodiment of the invention, the treatment of skin wrinkles is performed according to the second aspect of the invention.

According to a second aspect, the present invention provides a method for, improving the appearance, structure and/or function of skin in that a composition according to the first aspect of the invention is administered to the skin at the site of imperfection. In an embodiment of the invention, the mode of administration is intradermal injection of the composition.

The term "imperfection" refers to loss or absence of perfection. More specifically, "imperfections of Skin" refer to defects or flaw in the skin diminishing the appearance. Examples of "imperfections of skin" are wrinkles such as forehead wrinkles, anger wrinkles, worry wrinkles, slack eyelids, crow's feet, nasolabial wrinkles, and cellulite.

The method of treating skin imperfections, in particular wrinkles, comprises inter-and/or subdermal under-injection of the areas of skin affected. In an embodiment, the composition is injected through a needle having a size of no greater than 18 gauge, or an equivalent device. In one embodiment, the microparticles or beads comprising eukaryotic cells synthesizing and/or secreting constituents of the dermal extracellular matrix and being encapsulated in a biocompatible and/or biodegradeable sheath are injected by a syringe having a needle diameter of 26 gauge or less, i. e. 27 gauge, 28 gauge, 29 gauge, 30 gauge, 31 gauge, 32 gauge or even less, or other suitable techniques. The injection can be carried out either by multiple or several-fold injection into the areas of skin affected, only a very small volume being transferred at each puncture, until a total volume of from 0.1 ml to 2 ml has been under-injected. A planar cushioning and tightening of the skin can be achieved thereby, which leads to disappearance or partial disappearance of the wrinkles in the corresponding area.

Alternatively, the injection is carried out one to several times, a large volume being applied. In an embodiment, the injection in carried out by slow withdrawal of the injection needle with simultaneous under-injection of volume, this injection method being particularly suitable for deeper wrinkles. In one use form, barium-crosslinked beads of alginate having a diameter of 50-400 µm, in another embodiment having a diameter of about 100-250 µm are injected.

The invention of dermal under-injection with crosslinked alginates described here is suitable for skin deficiencies which are caused by e.g. ageing, environmental influences, weight loss, pregnancy, surgical interventions and acne. The use according to the invention is suitable in particular for treatment of wrinkles e.g. forehead wrinkles, anger wrinkles, worry wrinkles, slack eyelids, crows' feet, nasolabial wrinkles and for under-injection of lips and for treatment of wrinkles in the hand region, cellulite and decoltee.

Wrinkles that may be treated by employing the filler according to the instant invention include, but are not limited to, frown lines, medium depth wrinkles, such as nasolabial folds, lip augmentation, forehead wrinkles, glabellar lines, obvious mild to moderate nasal furrows and cheek wrinkles, crow's feet, perioral wrinkles, and acne scars.

In another embodiment of the present invention, the filler is for the treatment of, or for use in the treatment of, a medical condition, including lipoatrophy, gastroesophageal reflux disease (GERD), urine incontinence, vesico ureteral reflux (VUR), and the treatment of a psychological condition caused by the appearance of an aesthetic deficiency, including, but not limited to, frown lines, medium depth wrinkles, such as nasolabial folds, lip augmentation, forehead wrinkles, glabellar lines, obvious mild to moderate nasal furrows and cheek wrinkles, crow's feet, perioral wrinkles and acne scars.

In another embodiment of the present invention, the filler is for use in plastic, cosmetic, dental or general surgery, in ophthalmology, in orthopedics, for preventing tissue adhesions, or in urology.

According to the instant invention the filler may comprise a medium in which the beads are suspended. Said medium may be sterile water, phosphate-buffer saline (PBS), ringer solution, isotonic saline solution (0.9%), trometamol, citrate, carbonate, acetate, borate, amino acid, diethylamine, glucono delta lactone, glycine, lactate, maleic, methanesulfonic, monoethanolamine, tartrate buffer of choice or any combination thereof.

The filler as claimed in the instant invention may further comprise one or more active pharmaceutical ingredients selected from the group of anesthetics, analgenics, anti-microbials, anti-inflammatory drugs, growth factors, hormones, cosmeceuticals, vitamins, nutrients, stimulants, steroids, vasoconstrictors, anti-thrombotic agents, anti-coagulation agents, tranquilizers, muscle relaxants, antifungals, lipolytic agents and biorejunevation agents.

The term "active pharmaceutical ingredient" refers to all structures, which are pharmacologically active, thus resulting in a pharmacological effect in mammal and all known chemical forms thereof. Examples are, but not limited to, conjugates, isomers, esters, derivatives, metabolites, residues, salts or prodrugs thereof.

Anesthetics may be, but are not limited to, local anesthetics based on esters (Procaine, Benzocaine, Chloroprocaine, Cocaine, Cyclomethycaine, Dimethodcaine, Larocaine, Propoxycaine, Proparacaine, Tretracaine) or local anesthetics based on amides (Lidocaine, Articaine, Bupivacaine, Carticaine, Cinchocaine, Etidocaine, Levobupivacaine, Mepivacaine, Piperocaine, Prilocaine, Ropivacaine, Trimecaine). A suitable concentration for the anesthetic is from about 0.01 % to 6% based on the total weight of the composition and the agent selected.

Analgenics may be, but are not limited to, paracetamol, ibuprofen, diclofenac, naproxen, aspirin, celecoxib, etoricoxib, lumiracoxib, parecoxib, rofecoxib, valdecoxib, nimesulid, oxicams, such as piroxicam, isoxicam, tonexicam, sudoxicam, and CP-14,304; the salicylates, such as salicylic acid, aspirin, disalcid, benorylate, trilisate, safapryn, solprin, diflunisal, and fendosal; the acetic acid derivatives, such as diclofenac, fenclofenac, indomethacin, sulindac, tolmetin, isoxepac, furofenac, tiopinac, zidometacin, acematacin, fentiazac, zomepiract, clidanac, oxepinac, and felbinac; the fenamates, such as mefenamic, meclofenamic, flufenamic, niflumic, and tolfenamic acids; the propionic acid derivates, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and the pyrazoles, such as phenybutazone, oxyphenbutazone, feprazone, azapropazone, and trimethazone.

Antimicrobials may be, but are not limited to, antibiotics (amikacin, gentamycin, neomycin, tobramycin, kanamycin, meropenem, imipenem, cefaclor), antivirals (abacavir, aciclovir, amantadine, boceprevir, cidofovir, darunavir, edoxudine, famciclovir, ganciclovir, imunovir, inosine, interferon, lamivudine, nexavir, oseltamivir, penciclovir, ribavirin, rimantadine, viramidine, zidovudine) and antifungals (Miconazole, ketoconazole, itraconazole, clotrimazole, econazole, fluconazole, voriconazole, abafungin, naftifine, caspofungin, micafungin, benzoic acid, griseofulvin).

Anti-inflammatory drugs may be, but are not limited to, zinc salts, including zinc salts of polysaccharide acids, such as hyaluronic acid.

In one embodiment of the instant invention, the one or more active pharmaceutical ingredients are entrapped in the beads.

Antioxidants may be, but are not limited to, vitamin E, vitamin C, glutathione, coenzyme Q, resveratrol, bisulfite sodium, butylated hydroxyl anisole/toluene, cysteinate, dithionite sodium, gentisic acid, glutamate, formaldehyde sulfoxylate sodium, metabisulfite sodium, monothiogylcerol, propyl gallate, sulfite sodium, thiogycolate sodium, flavonoids, catalase, lycopene, carotenes, lutein, superoxide dismutase and peroxidises, Zinc or mixtures thereof.

The invention will be explained in more detail by virtue of the examples set forth herein below. While at least one exemplary embodiment is presented, it should be appreciated that a vast number of variations exist. It should also be appreciated that the examplatory embodiments are only examples, and are not intended to limit the scope, applicability, or configuration in any way. Rather, the forgoing description will provide those with ordinary skill in the art with a convenient road map for implementing at least one exemplary embodiment, it being understood that various changes may be made without departing from the scope as set forth in the appended claims.

### Examples

### Example 1: Preparation of an alginate solution

A 0.6% (w/v) alginate solution is prepared from sterile reagents and materials. All steps are performed under a laminar flow. A sterile 50 ml polystyrene tube is tared. 0.15 g of dried alginate as the solid substance is introduced into the tube with a sterile spatula. 25 ml of 0.9% NaCl solution are added with sterile pipette. The tube is closed and gently rotated until the alginate has dissolved completely. The alginate solution prepared in this way is ready for preparing alginate beads.

### Example 2: Preparation of a suspension of human dermal fibroblasts

Human dermal fibroblasts are obtained according to standard protocols from culture or from natural sources. The fibroblasts are harvested by centrifugation, and are resuspended in an aliquot of the alginate solution obtained from example 1.

### Example 3: Preparation of a barium-containing precipitation bath

4.48 g BaCl₂, 0.77 g histidine, 7.25 g NaCl and 1,000 ml of sterile distilled water are mixed under sterile conditions and the mixture is stirred until all substances are dissolved. The pH of the resulting solution is adjusted to pH 7 ±.0.1 with NaOH or HCl. The osmolality of the solution prepared is checked with a freezing point osmometer, and must be 290 mOsmol ± 3 mOsmol. The solution is then sterilized and can be used for preparing barium-crosslinked alginate beads.

### Example 4: Preparation of Barium-crosslinked Algiante Beads including human dermal fibroblasts

The cell suspension (see Example 2) is collected in or transferred to a sterile 50 ml centrifuge tube. A sterilized encapsulating apparatus is fixed with 2 screws at a height of 10 ± 2 cm to an electrically regulatable syringe advancer. A compressed air hose is connected to the apparatus with a clamp and the compressed air is then regulated to a value of 3 ± 1.0 l/min. An open Petri dish is placed under the drop formation nozzle. Three portions of 1 ± 0.1 ml each of sterile water are flushed through the nozzle channel with a 1 ml syringe for cleaning. The speed of the advancers is adjusted to 3 ±.0.5 units. A sterile 1 ml syringe is filled with the centrifuged alginate solution without air bubbles and fitted on to the nozzle. In order to prepare beads having a diameter of 200 µm, the diameter of the channel of the drop formation nozzle must be approx. 100 µm. The nozzle advancer is switched on and a new Petri dish containing 40 ml of precipitating bath (see Example 3) is placed under the nozzle. Drops of alginate containing human dermal fibroblasts form at the end of the nozzle. The droplets are torn off by a stream of air and fall into the precipitating bath. The droplets are cured in the precipitating bath for 10 minutes to form beads. When the alginate beads have cured, they are collected and washed 5 times with 0.9% NaCl.

For preparing alginate beads, the JetCutter device (geniaLab, Braunschweig, Germany) can be used as well. A viscous alginate solution containing the desired amount of cells is prepared. A solid jet of alginate solution including the cells comes out of a nozzle and is cut by rotating wires into initially cylindrical segments. The segments then form beads due to surface tension on their way to a hardening device. The segments fall into the precipitating bath to be cured for 10 minutes.

### Example 5: Detection of collagen

The alginate beads obtained in Example 4 are transferred into an appropriate amount of culture medium (e.g. MEM + 10% FCS) for propagating the fibroblasts. The supernatant of the culture medium is collected after 1 day, 2 days, 3 days, 4 days up to two weeks and then tested for collagen being secreted into the medium by means of, for example, Western-Blot analysis, ELISA, etc. utilizing monoclonal anti-collagen antibodies according to standard procedures.

## Claims

1. Composition for use as a dermal filler comprising:
an alloplastic injectable suspension comprising beads based on a biocompatible material and a physiologically acceptable suspending agent, wherein the beads include eukaryotic cells synthesizing and/or secreting one or more constituents of the dermal extracellular matrix, said eukaryotic cells being encapsulated by a sheath of said biocompatible material.

2. Composition according to claim 1, **characterized in that** the biocompatible material is selected from the group consisting of polylactide, chondroitin sulfate, polyesters, polyethylene glycols, polycarbonates, polyvinyl alcohols, polyacrylamides, polyamides, polyacrylates, polyetheresters, polymethacrylates, polyurethanes, polycaprolactone, polyphophazenes, polyorthoesters, polyglycolides, copolymers of lysine and lactic acid, copolymers of lysine-RGD and lactic acid, chitosan, alginates, collagens, hyaluronic acid, pectin, gellan, carrageenan and combinations thereof.

3. Composition according to claim 1 or 2, **characterized in that** the biocompatible material is selected from the group consisting of calcium alginate, barium alginate and combinations thereof.

4. Composition according to any one of the preceding claims, **characterized in that** the sheath comprises pores having a diameter of - in average - at least 10 nm and not exceeding 300 nm.

5. Composition according to any one of the preceding claims, **characterized in that** the eukaryotic cells synthesize glycosaminoglycans.

6. Composition according to any one of the preceding claims, **characterized in that** the eukaryotic cells secrete at least one proteoglycan.

7. Composition according to claim 6, **characterized in that** the proteoglycan is selected from the group consisting of aggrecan, betaglycan, decorin, perlecan, serglycin and syndecan-l.

8. Composition according to any one of the preceding claims, **characterized in that** the eukaryotic cells secrete a protein selected from the group consisting of collagen and elastin and precursors thereof.

9. Composition according to claim 8, **characterized in that** the collagen is a collagen selected from the group consisting of collagen type I, collagen type III, collagen type IV and collagen type V and collagen precursors.

10. Composition according to any one of the preceding claims, **characterized in that** the beads have a diameter of about 10 µm to about 2,000 µm.

11. Composition according to any one of the preceding claims, **characterized in that** the beads contain from about 40 to about 1,000 eukaryotic cells.

12. Use of a composition according to any one of the preceding claims as dermal filler for improving imperfections of the skin.

13. Method for improving imperfections of skin, **characterized in that** a composition according to any one of claims 1 to 11 is intradermally and/or subdermally under-injected at the area of skin affected.
